# EUROPÄISCHE PATENTSCHRIFT

(11) **EP 2 881 129 B1**
(45) Veröffentlichungstag und Bekanntmachung des Hinweises auf die Patenterteilung: **01.05.2019**
(21) Anmeldenummer: 14189985.6
(22) Anmeldetag: 23.10.2014
(51) Int. Cl.: A61M 5/00, A61M 5/168, A61M 5/172, A61M 5/142

(54) **Injektor zur Injektion eines Fluids und Verfahren zur Steuerung eines Injektors**
Injector for injecting a fluid and method for controlling an injector
Injecteur destiné à l'injection d'un fluide et procédé de commande d'un injecteur

(30) Priorität: 03.12.2013 DE 102013113387
(43) Veröffentlichungstag der Anmeldung: 10.06.2015
(73) Patentinhaber: Ulrich GmbH & Co. KG, 89081 Ulm (DE)
(72) Erfinder: Seibold, Felix, 89081 Ulm (DE)
(74) Vertreter: Charrier Rapp & Liebau

(56) Entgegenhaltungen:
- US-A1- 2005 230 575
- US-A1- 2009 076 461
- US-A1- 2012 078 218

## Beschreibung

Die Erfindung betrifft einen Injektor nach dem Oberbegriff des Anspruchs 1 sowie ein Verfahren zur Steuerung eines Injektors nach dem Oberbegriff des Anspruchs 2.

Aus der US 6,673,033 B1 sind Injektorsysteme zur Injektion eines Fluids in medizinischen Injektionsverfahren und Verfahren zu deren Steuerung bekannt, wobei das Injektorsystem einen Antriebsmechanismus, mit dem ein zu injizierendes Fluid unter Druck gesetzt wird, und einen Sensor zur Erfassung einer Variable aufweist, die proportional zum Fluiddruck ist und einen Drucküberwachungsmechanismus umfasst, der in Verbindung mit dem Sensor und dem Antriebsmechanismus steht und den Injektionsvorgang abbricht, wenn der Sensor einen Wert der erfassten Variable misst, welcher einem Gefährdungsdruck (pressure hazard limit) entspricht. Um zu verhindern, dass der Fluiddruck sich dem Gefährdungsdruck annähert oder diesen übersteigt, ist in dem Drucküberwachungsmechanismus weiterhin vorgesehen, dass die Antriebsleistung des Antriebs auf einen vorgegebenen Grenzwert beschränkt wird, wenn die dem Fluiddruck proportionale Variable einen Wert erreicht, der einer vorgegebenen Druckgrenze (power limiting pressure) entspricht, wobei diese vorgegebene Druckgrenze unterhalb des Gefährdungsdrucks liegt. Der Druck des zu injizierenden Fluids wird dabei entweder mittelbar, beispielsweise über den Strom eines Elektromotors des Antriebsmechanismus, oder unmittelbar über einen Kraft- oder Druckaufnehmer erfasst, wobei die unmittelbare Erfassung des Fluiddrucks über einen Kraft- oder Druckaufnehmer bevorzugt wird, weil diese genauer ist und etwaige Toleranzen des Injektorsystems ausgleicht. Der Drucküberwachungsmechanismus ist so eingerichtet, dass er die von einem Bediener des Injektionssystems vorgegebenen Parameter, mit dem das Injektionssystem betrieben werden soll, aufhebt und die Antriebsleistung des Antriebsmechanismus reduziert oder vollständig abstellt, wenn der erfasste Fluiddruck oberhalb der vorgegebenen Druckgrenze für die Leistungsreduzierung (power limiting pressure) liegt oder den Gefährdungsdruck (pressure hazard limit) überschreitet. Durch die Leistungsreduzierung der Antriebsleistung bei Überschreiten des erfassten Fluiddrucks über die vorgegebene Druckgrenze der Leistungsreduzierung (power limiting pressure), kann verhindert werden, dass der Gefährdungsdruck (pressure hazard limit) überhaupt erreicht wird. Sollte der Gefährdungsdruck dennoch erreicht oder sogar überschritten werden, wird die Leistungszufuhr des Antriebsmechanismus vollständig abgestellt, um einen weiteren Druckanstieg und damit eine drohende Gefährdung des Patienten oder Schäden am Injektionssystem zu vermeiden.

Die US 2009/0076461 A1 zeigt eine Steuer- und Kommunikationseinrichtung für ein Infusionsgerät, mit der eine Pumpe ferngesteuert und -überwacht werden kann.

Dieses bekannte Injektionssystem und das Verfahren zu dessen Steuerung erweisen sich jedoch als nachteilig, weil kurzzeitige Anstiege des Fluiddrucks, die beispielsweise beim Anlaufen des Injektionssystems zu Beginn des Injektionsvorgangs auftreten können, zu einer unnötigen Leistungsreduzierung der Antriebsleistung des Antriebsmechanismus oder bei sehr hohen kurzzeitigen Druckspitzen sogar zum vollständigen Abschalten des Antriebsmechanismus und damit zu einer Unterbrechung des Injektionsvorgangs führen kann. Dies führt zu einer Verlängerung der Injektionszeit, in der dem Patienten eine vorgegebene Menge (Dosierung) des zu injizierenden Fluids zugeführt werden soll. Bei einem Abbruch des Injektionsvorgangs ist eine Überprüfung des Systems durch den Bediener und ein Neustart des Injektionsvorgangs notwendig, was ebenfalls zu einer unerwünschten Verlängerung der Dauer des Injektionsvorgangs und zu einer Reduzierung des Durchsatzes bei einer Mehrzahl von mit dem Injektionssystems hintereinander auszuführenden Injektionsvorgängen führt.

Hiervon ausgehend liegt der Erfindung die Aufgabe zugrunde, einen gattungsgemäßen Injektor und ein Verfahren zu dessen Steuerung bereit zu stellen, mit denen einerseits ein Überschreiten eines Gefährdungsdrucks in dem Injektionssystem zuverlässig verhindert werden kann, andererseits unnötige Verlängerungen des Injektionsvorgangs durch eine Leistungsreduzierung oder einer Abschaltung des Antriebsmechanismus vermieden werden können. Mit dem Injektor und dem Verfahren zu dessen Steuerung soll die Effizienz und der Durchsatz von mehreren, mit dem Injektor hintereinander auszuführenden Injektionsvorgängen erhöht werden, bei gleichzeitig gegebener Sicherheit, dass ein vorgegebener Grenzwert für den Druck des zu injizierenden Fluids in dem Injektionssystem nicht überschritten wird.

Diese Aufgaben werden mit dem Injektor mit den Merkmalen des Anspruchs 1 und dem Verfahren mit den Merkmalen des Anspruchs 2 gelöst. Bevorzugte Ausführungsbeispiele des Injektors und des Verfahrens sind den abhängigen Ansprüchen zu entnehmen.

Bei dem erfindungsgemäßen Injektor ist der Antriebsmechanismus zur Förderung eines zu injizierenden Fluids in den menschlichen oder tierischen Körper mit einer vorgegebenen Durchflussrate oder Fließgeschwindigkeit durch eine Pumpe bereitgestellt. Zum Einstellen einer gewünschten Durchflussrate (Volumen des zu injizierenden Fluids pro Zeiteinheit) oder Fließgeschwindigkeit (Geschwindigkeit des zu injizierenden Fluids im Injektionssystem, insbesondere in den Injektionsschläuchen), welche vom Bediener des Injektors vorgegeben werden kann, wird die Pumpe von einer Steuereinrichtung gesteuert. An die Steuereinrichtung ist eine Druckmesseinrichtung zur Erfassung des Drucks des von der Pumpe geförderten Fluids gekoppelt. Der Bediener des Injektors gibt der Steuereinrichtung einen Solldruck des zu injizierenden Fluids in dem Injektionssystem vor und die Druckmesseinrichtung erfasst den tatsächlichen Druck (Ist-Druck) des Fluids an einer vorgegebenen Stelle im Injektionssystem, wobei die Erfassung des Fluiddrucks entweder unmittelbar, beispielsweise durch einen Kraft- oder Druckaufnehmer, oder mittelbar durch Erfassung eines Messwerts einer Größe, die zum Druck des Fluids in dem Injektionssystem proportional ist, erfolgen kann.

Der auf diese Weise erfasst Druck des Fluids (Ist-Druck) wird in der Steuereinrichtung mit dem vorgegebenen Solldruck verglichen. Wenn der erfasste Druck des Fluids (Ist-Druck) den vorgegebenen Solldruck während eines bestimmten Zeitraums überschreitet, wird der zeitliche Druckverlauf des von der Druckmesseinrichtung erfassten Drucks (Ist-Druck) über diesen Zeitraum zur Ermittlung eines Druckintegrals integriert und die Pumpe wird (erst) dann abgeschaltet, wenn das so ermittelte Druckintegral einen vorgegebenen Integralgrenzwert überschreitet. Der von der Druckmesseinrichtung (unmittelbar oder mittelbar) erfasste Druck des Fluids (Ist-Druck) wird also über die Zeit integriert, wenn und solange der erfasste Fluiddruck über dem vorgegebenen Solldruck liegt. Durch die Integration des zeitlichen Verlaufs des erfassten Drucks des Fluids (Ist-Druck) über den Zeitraum, in dem der erfasste Druck über dem Solldruck liegt, werden etwaig auftretende Druckspitzen unterdrückt. Eine Abschaltung der Pumpe erfolgt nur dann, wenn das von der Steuereinrichtung berechnete Druckintegral den vorgegebenen Integralgrenzwert überschreitet. Dies wird regelmäßig nur dann der Fall sein, wenn der erfasste Druck des Fluids (Ist-Druck) über einen längeren Zeitraum oberhalb des vorgegebenen Solldrucks ist, weshalb ggf. kurzzeitig auftretende Druckspitzen, welche in der Regel ungefährlich für den Patienten sind, unterdrückt werden und nicht zu einem Abschalten der Pumpe führen. Auf diese Weise kann ein unnötiges Abschalten der Pumpe oder eine Leistungsreduzierung des Antriebsmechanismus, die zu einer unnötigen Verlängerung des Injektionsvorgangs führen würden, vermieden werden.

Der vorgegebene Integralgrenzwert wird zweckmäßig so berechnet und eingestellt, dass der tatsächliche Druck des Fluids in dem Injektionssystem einen möglichen Gefährdungsdruckwert nicht erreichen oder überschreiten kann. Auf die Vorgabe eines oberen Grenzwerts für den Druck des Fluids im Injektionssystem, der einem Gefährdungsdruck-Grenzwert entspricht, kann dadurch verzichtet werden. Aus Sicherheitsgründen kann jedoch ein solcher oberer Grenzwert zusätzlich vorgegebenen und die Steuerung der Pumpe so eingestellt werden, dass die Pumpe in jedem Fall abgeschaltet wird, wenn der von der Druckmesseinrichtung erfasste Druck des Fluids (Ist-Druck) den oberen Grenzwert, der einem Gefährdungsdruck entspricht, erreicht oder sogar überschreitet.

Zur Ermittlung des Druckintegrals wird zweckmäßig der Differenzdruck zwischen dem von der Druckmesseinrichtung erfassten Druck (Ist-Druck) und dem Solldruck über den Zeitraum integriert, in dem der erfasste Druck (Ist-Druck) über dem Solldruck liegt.

In einem bevorzugten Ausführungsbeispiel des erfindungsgemäßen Injektors bzw. des erfindungsgemäßen Steuerverfahrens ist die Steuereinrichtung so eingerichtet, dass sie den von der Druckmesseinrichtung erfassten Druck des Fluids (Ist-Druck) mit dem Solldruck vergleicht und bei Überschreiten des Solldrucks die Leistung der Pumpe reduziert, bis der tatsächliche Druck des Fluids (Ist-Druck) wieder dem Solldruck entspricht. Auf diese Weise kann verhindert werden, dass der tatsächliche Druck des Fluids in dem Injektionssystem über einen längeren Zeitraum oberhalb des Solldrucks liegt, was in Abhängigkeit des vorgegebenen Integralgrenzwerts zu einem Abschalten der Pumpe führen könnte.

Diese und weitere Vorteile und Merkmale der Erfindung ergeben sich aus dem nachfolgend unter Bezugnahme auf die begleitenden Zeichnungen näher beschriebenen Ausführungsbeispiel. Die Zeichnungen zeigen:
- **Figur 1:**: Schematische Darstellung der Bestandteile eines erfindungsgemäßen Injektors;
- **Figur 2:**: Vorderansicht eines konkreten Ausführungsbeispiels eines erfindungsgemäßen Injektors;
- **Figur 3:**: Das in dem Injektor von Figur 2 verwendete Schlauchsystem mit einem Injektionsschlauch und einer Druckmesseinrichtung;
- **Figur 4:**: Diagramme mit dem zeitlichen Verlauf des Drucks und der Durchflussrate (flow) des in dem Injektionsschlauch von Figur 3 geförderten Fluids bei Durchführung des erfindungsgemäßen Verfahrens zur Steuerung des Injektors von Figur 2.

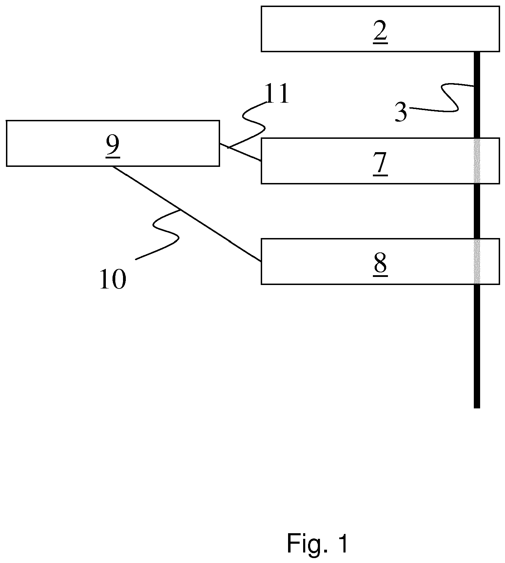

In **Figur 1** sind die Bestandteile eines erfindungsgemäßen Injektors schematisch in einem Blockdiagramm dargestellt. Der Injektor 1 kann beispielsweise zur Injektion eines Fluids, insbesondere eines flüssigen Kontrastmittels für Röntgen- oder kernspintomographische Untersuchungen in den menschlichen oder tierischen Körper verwendet werden. Die Injektion des zu injizierenden Fluids erfolgt dabei in der Regel intravenös. Das zu injizierende Fluid wird in einem Fluidreservoir 2 bevorratet. An das Fluidreservoir 2 ist ein Injektionsschlauch 3 angeschlossen. Der Injektionsschlauch 3 ist mit einer Pumpe 7 gekoppelt. Über die Pumpe 7 wird das im Fluidreservoir 2 bevorratete Fluid durch den Injektionsschlauch 3 gefördert. Bevorzugt handelt es sich bei der Pumpe 7 um eine Schlauchpumpe, in welche der Injektionsschlauch 3 eingelegt wird, so dass die Schlauchpumpe das Fluid aus dem Fluidreservoir 2 durch den Injektionsschlauch 3 fördern kann. Dadurch wird auch sicher gestellt, dass die Pumpe nicht mit den zu fördernden Fluid in Kontakt kommt, was die Sterilität des Injektionsvorgangs gewährleistet.

Stromabwärts zur Pumpe 7 ist eine Druckmesseinrichtung 8 in bzw. an dem Injektionsschlauch 3 angeordnet. Mit der Druckmesseinrichtung 8 kann der Druck des in dem Injektionsschlauch strömenden Fluids erfasst werden. Die Druckmesseinrichtung 8 steht über eine Datenverbindung 10 mit einer Steuereinrichtung 9 in Verbindung. Die Steuereinrichtung 9 ist über eine weitere Datenverbindung 11 mit der Pumpe 7 gekoppelt, um die Pumpe 7 zu steuern. Die Pumpe 7 wird dabei zweckmäßig von einem Elektromotor angetrieben und über die Steuereinrichtung 9 elektrisch angesteuert. Bei den Datenleitungen 10, 11 kann es sich um kabelgebundene oder auch um drahtlose Datenverbindungen handeln.

Die Steuereinrichtung 9 weist zweckmäßig eine hier nicht dargestellte Eingabeeinrichtung auf, mit der ein Bediener des Injektors 1 gewünschte Parameter (Soll-Parameter) zur Steuerung des mit dem Injektor 1 durchzuführenden Injektionsvorgangs eingeben kann. Der Bediener kann über die Eingabeeinrichtung beispielsweise einen Solldruck sowie eine gewünschte Durchflussrate (Soll-Flow) des Fluids durch den Injektionsschlauch 3 vorgeben.

Unter Durchflussrate (flow-rate) wird hierbei das Volumen des Fluids verstanden, welches pro Zeiteinheit an einer vorgegebenen Stelle (beispielsweise am Ende) des Injektionsschlauchs 3 strömt. Alternativ zur Durchflussrate (Volumen pro Zeiteinheit) kann auch eine Durchflussgeschwindigkeit (Strömungsgeschwindigkeit des Fluids) oder auch eine Strömungsdichte vorgegeben werden. Die vom Bediener über die Eingabeeinrichtung eingegebenen Soll-Werte für den Druck und die Durchflussrate des Fluids werden in einem Speicher der Steuereinrichtung 9 gespeichert, so dass eine Datenverarbeitungseinrichtung, die Bestandteil der Steuereinrichtung 9 ist, auf die in dem Speicher hinterlegten Soll-Werte (SollDruck und Soll-Flow) zugreifen kann.

Um eine korrekte Dosierung des zu injizierenden Fluids zu gewährleisten, wird die Pumpe 7 bevorzugt von der Steuereinrichtung 9 so gesteuert, dass eine konstante Durchflussrate des Fluids über einen vorgegebenen Injektionszeitraum oder auch gepulste Fluid-Boli von der Pumpe 7 durch den Injektionsschlauch 3 gefördert werden. In Abhängigkeit der Eigenschaften und Parameter der Pumpe 7 und des verwendeten Injektionsschlauchs 3 können sich dabei, auch zu verschiedenen Zeitpunkten während der Injektionsdauer, unterschiedliche Druckwerte des von der Pumpe 7 durch den Injektionsschlauch 3 geförderten Fluids in dem Injektionsschlauch 3 ausbilden. Zur Vermeidung eines Überdrucks steuert die Steuereinrichtung 9 die mit ihr gekoppelte Pumpe 7 gemäß dem erfindungsgemäßen Verfahren, welches nachfolgend noch im Einzelnen erläutert wird.

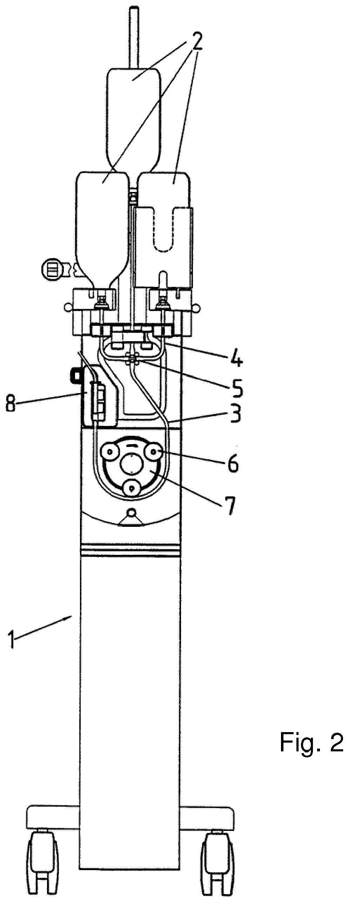

In **Figur 2** ist ein bevorzugtes Ausführungsbeispiel eines erfindungsgemäßen Injektors 1 in einer Vorderansicht gezeigt. Der Injektor 1 umfasst das Fluidreservoir 2, einen an das Fluidreservoir 2 angeschlossenen Injektionsschlauch 3, eine als Schlauchpumpe (Peristaltikpumpe) ausgebildete Pumpe 7 sowie eine an dem Injektionsschlauch 3 stromabwärts der Pumpe 7 angeordnete Druckmesseinrichtung 8 zur Erfassung des Fluiddrucks im Injektionsschlauch 3. Das Fluidreservoir 2 ist bei dem in Figur 2 gezeigten Ausführungsbeispiel von drei verschiedenen Vorratsbehältern gebildet, welche jeweils durch Absperrventile oder abklemmbare Verbindungsschläuche 4 und über ein Verzweigungsstück 5 mit dem Injektionsschlauch 3 verbunden sind. In den Vorratsbehältern des Fluidreservoirs 2 sind die zu injizierenden Fluide bevorratet. Dabei kann es sich beispielsweise um verschiedene Kontrastmittel-Flüssigkeiten sowie um eine Spüllösung (beispielsweise eine NaCl-Lösung) handeln. Die Absperrventile in den Verbindungsschläuchen 4 sind dabei zweckmäßig mit der Steuereinrichtung 9 gekoppelt und können von der Steuereinrichtung 9 geöffnet bzw. geschlossen werden, so dass von einem Bediener des Injektors 1 über die Steuereinrichtung 9 ausgewählt werden kann, welches in den einzelnen Vorratsbehältern des Fluidreservoirs 2 bevorratete Fluid durch den Injektionsschlauch 3 gefördert werden soll. Das freie Ende des Injektionsschlauchs 3 verfügt zweckmäßig über einen genormten Schlauchanschluss (beispielsweise einen Luer-Anschluss), über den an den Injektionsschlauch 3 ein Patientenschlauch angeschlossen werden kann, an dessen Ende eine Injektionskanüle vorgesehen ist, welche dem Patienten intravenös appliziert werden kann. Das zu injizierende Fluid wird dann von der Pumpe 7 durch den Injektionsschlauch 3 und in den daran angeschlossenen Patientenschlauch gefördert und auf diese Weise dem Patienten intravenös injiziert.

Die als Schlauchpumpe ausgebildete Pumpe 7 weist in dem in Figur 2 gezeigten Ausführungsbeispiel drei Quetschrollen 6 und ein zugeordnetes Gegenlager auf. Der Injektionsschlauch 3 wird so in die Schlauchpumpe 7 eingelegt, dass die Quetschrollen 6 den Injektionsschlauch 3 gegen das Gegenlager drücken und dadurch das Fluid in dem Schlauch unter Quetschung des Schlauchs in einer Förderrichtung mit einer vorgebbaren Durchflussrate (flow rate) fördern.

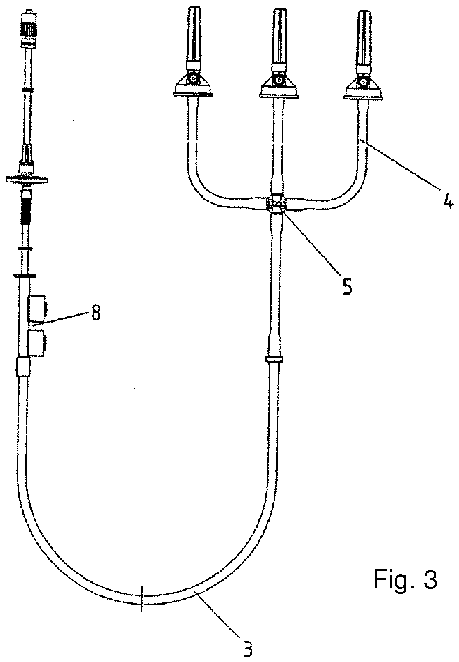

Das in dem Injektor 1 von Figur 2 gezeigte Schlauchsystem mit dem Injektionsschlauch 3 und den Verbindungsschläuchen 4, welche über das Verzweigungsstück 5 mit einem Ende des Injektionsschlauchs 3 verbunden sind, ist in **Figur 3** im Detail dargestellt. Die freien Enden der Verbindungsschläuche 4 werden jeweils mit einem Vorratsbehälter des Fluidreservoirs 2 verbunden und der Injektionsschlauch 3 wird - wie oben dargestellt - in die Schlauchpumpe eingeführt. Wie aus Figur 3 weiterhin ersichtlich, ist stromabwärts der Pumpe 7 eine Druckmesseinrichtung 8 an dem Injektionsschlauch 3 angeordnet. Bei der Druckmesseinrichtung 8 handelt es sich zweckmäßig um ein Druckmessgerät mit einer Druckkammer und einer Membran, welche mit einem Drucksensor gekoppelt ist. Die Druckkammer steht dabei über Öffnungen in der Wandung des Injektionsschlauch 3 mit dem Inneren des Injektionsschlauchs 3 in Verbindung, so dass der Druck des in dem Injektionsschlauch 3 strömenden Fluids in die Druckkammer und von dort auf die Membran übertragen wird. Über die Kopplung der Membran an den Drucksensor kann dieser den im Innern des Injektionsschlauchs 3 herrschenden Druck, der durch das durchströmende Fluid hervorgerufen wird, erfassen. Der von der Druckmesseinrichtung 8 erfasste Fluiddruck (Ist-Druck) wird über die in Figur 1 dargestellte Datenverbindung 10 an die Steuereinrichtung 9 übermittelt und dort in einer Datenverarbeitungseinrichtung ausgewertet.

Die in der Steuereinrichtung 9 enthaltene Datenverarbeitungseinrichtung vergleicht den von der Druckmesseinrichtung erfassten Druck (Ist-Druck p) und vergleicht diesen mit dem vorgegebenen Solldruck, der im Datenspeicher der Steuereinrichtung 9 hinterlegt ist. Sobald der von der Druckmesseinrichtung erfasste Druck (Ist-Druck p) den vorgegebenen Solldruck überschreitet, integriert die Datenverarbeitungseinrichtung den zeitlichen Druckverlauf p(t) des Ist-Drucks (also des von der Druckmesseinrichtung erfassten Drucks p) über den Zeitraum Δt = t₂ - t₁ auf, in dem der Ist-Druck oberhalb des Solldrucks liegt. Das auf diese Weise ermittelte Druckintegral I(t) = ∫ p(t)dt wird während des Zeitraums Δt, in dem der Ist-Druck größer als der Solldruck ist, ständig mit einem vorgegebenen Integralgrenzwert Iₘₐₓ verglichen. Wenn das so ermittelte Druckintergral I den vorgegeben und im Datenspeicher der Steuereinrichtung 9 hinterlegten Intergralgrenzwert Iₘₐₓ überschreitet (also I(t) > Iₘₐₓ), wird die Pumpe 7 abgeschaltet oder zumindest mit geringerer Leistung betrieben, bis der von der Druckmesseinrichtung erfasste Druck (Ist-Druck p) wieder dem Solldruck entspricht.

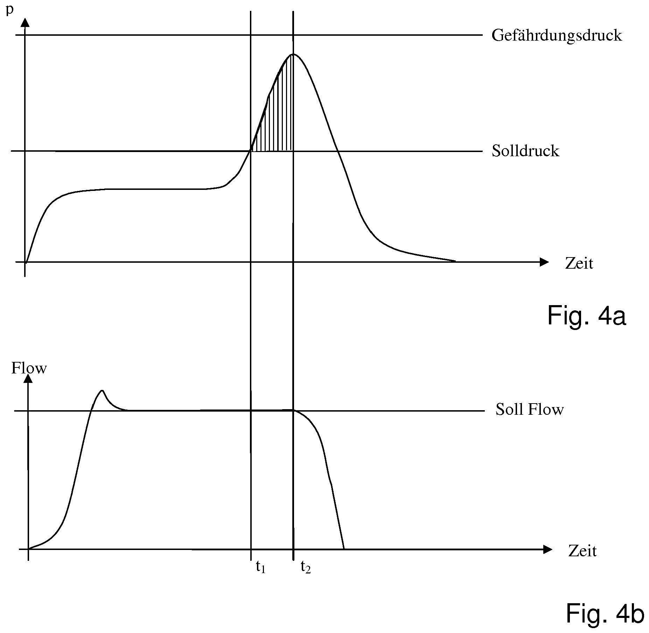

Der sich bei dieser Steuerung des Injektors ergebende zeitliche Druck- und Durchflussraten-Verlauf ist in **Figur 4** dargestellt, wobei Figur 4a den zeitlichen Druckverlauf (p(t)) und Figur 4b den zugehörigen zeitlichen Verlauf der Durchflussrate (flow rate) zeigt. Zu Beginn des Injektionsvorgangs (t=0) wird die Pumpe 7 eingeschalten und fördert aus dem Fluidreservoir 2 das zu injizierende Fluid durch den Injektionsschlauch 3. Dabei steigt der Druck des Fluids in dem Injektionsschlauch rasch an, bis ein gewünschter Wert der Durchflussrate (Flow rate) erreicht wird. Die tatsächliche Durchflussrate, deren zeitlicher Verlauf in Figur 4b gezeigt ist, nähert sich dabei - in der Regel nach einem Einschwungpeak - der vorgegebenen Soll-Durchflussrate (Soll-Flow) an. Die Steuereinrichtung 9 steuert die Leistung der Pumpe 7 zweckmäßig derart, dass die tatsächliche Durchflussrate der vorgegebenen Soll-Durchflussrate (Soll-Flow) entspricht. Falls sich - beispielsweise durch eine Fehlfunktion der Pumpe 7 oder eine Verstopfung im Injektionsschlauch 3 - ein Fluidstau ergeben sollte, der zu einem unerwünschten Ansteigen des tatsächlichen Fluiddrucks in dem Injektionsschlauch führen sollte, wird die Leistung der Pumpe 7 entsprechend dem erfindungsgemäßen Verfahren abgeregelt oder vollständig abgestellt, um ein weiteres Ansteigen des Fluiddrucks und insbesondere einen Gefährdungsdruck im Injektionsschlauch 3 zu vermeiden, der zu Beschädigungen im Injektor oder zu Schäden im Gewebe des Patienten führen könnte. Eine solche Situation ist im zeitlichen Verlauf des Fluiddrucks und der Durchflussrate in den Figuren 4a und 4b im Zeitraum Δt zwischen den Zeitpunkten t₁ und t₂ dargestellt. Zum Zeitpunkt t₁ übersteigt der von der Druckmesseinrichtung 8 erfasste Druck (Ist-Druck p) den vorgegebenen und in dem Datenspeicher der Steuereinrichtung 9 hinterlegten Solldruck. Sobald der Solldruck überschritten wird, beginnt die Datenverarbeitungseinrichtung damit, den zeitlichen Verlauf des Ist-Drucks p(t) zu integrieren, solange der Ist-Druck oberhalb des Solldrucks liegt. Der durch die zeitliche Intergration des Ist-Drucks ermittelte Wert des Druckintergrals I(t) entspricht dabei der Fläche unter der Druckkurve des zeitlichen Verlaufs des Ist-Drucks p(t). Zweckmäßig wird - wie in Figur 4a angedeutet - der Differenzwert zwischen dem Ist-Druck p(t) und dem vorgegebenen Solldruck zeitlich integriert, so dass das ermittelte Druckintergral I(t) der Fläche zwischen der Solldruck-Linie und der Druckkurve des Ist-Drucks p(t) während des Zeitraums Δt entspricht, in dem der Ist-Druck p(t) oberhalb des vorgegebenen Solldrucks liegt. Sobald der so ermittelte Wert des Druckintegrals I einen vorgegeben und in dem Datenspeicher hinterlegten Intergralgrenzwert Iₘₐₓ überschreitet, wird die Pumpe 7 von der Steuereinrichtung 9 entweder vollständig abgestellt oder nur noch mit geringerer Pumpleistung betrieben. Dadurch wird ein weiteres Ansteigen des Ist-Drucks vermieden und der zeitliche Verlauf des Ist-Drucks fällt zunächst auf einen Wert unterhalb des Solldrucks ab und - bei vollständigem Stillsetzen der Pumpe 7 - wird im weiteren zeitlichen Verlauf auf 0 reduziert. Sobald die Pumpe 7 entweder komplett stillgesetzt oder mit verminderter Pumpleistung betrieben wird, sinkt auch die tatsächliche Durchflussrate ab, was in Figur 4b im Bereich oberhalb des Zeitpunkts t₂ ersichtlicht ist. Bei vollständigem Stillsetzen der Pumpe 7 kommt der Durchfluss vollständig zum erliegen und reduziert sich - wie in Figur 4b dargestellt, auf 0.

Durch diese Steuerung der Pumpe 7 durch die Steuereinrichtung 9 kann vermieden werden, dass der Ist-Druck p(t) über einen längeren Zeitraum oberhalb des vorgegebenen Solldrucks liegt. Der vorgegebene Solldruck wird dabei zweckmäßig so gewählt, dass er weit genug unterhalb eines möglichen Gefährdungsdrucks liegt, bei dem eine Beschädigung des Injektors 1 oder dessen Komponenten oder ein Verletzungsrisiko des Patienten vorliegt. Wenn der vorgegebene Solldruck weit genug unterhalb eines solchen Gefährdungsdrucks liegt, kann mit dem erfindungsgemäßen Steuerverfahren mit ausreichender Sicherheit gewährleistet werden, dass der in dem Injektionsschlauch 3 herrschende Druck den Gefährdungsdruck nicht erreicht, weil die Pumpe 7 vorher abgeschaltet oder zumindest mit geringerer Leistung betrieben wird. Eine zusätzliche Sicherung des Pumpenbetriebs, über die Vorgabe und Einstellung eines Gefährdungsdrucks ist zwar möglich, jedoch in dem erfindungsgemäßen Steuerverfahren nicht mehr zwingend notwendig. Insbesondere kann durch das erfindungsgemäße Steuerverfahren vermieden werden, dass bei nur kurzzeitig auftretenden Druckspitzen ein Abschalten oder eine Leistungsreduzierung der Pumpe erfolgt, welche durch Vermeidung des Gefährdungsdrucks nicht notwendig wäre und daher lediglich zu einer unnötigen Verzögerung des Injektionsvorgangs führen würde. Auf diese Weise kann der Durchsatz des Injektors mit dem erfindungsgemäßen Steuerungsverfahren erhöht werden.

Die Erfindung ist nicht auf die beschriebenen Ausführungsbeispiele beschränkt. So ist es z. B. möglich, den Druck des Fluids in dem Injektionsschlauch 3 auf andere Art, beispielsweise indirekt über den Motorstrom der Pumpe 7 zu erfassen. Weitere Änderungen des erfindungsgemäßen Injektors und des erfindungsgemäßen Steuerverfahrens ergeben sich für einen Fachmann durch fachübliche Kenntnisse und Maßnahmen und liegen im Schutzbereich der folgenden Ansprüche.

## Patentansprüche

1. Injektor zur Injektion eines Fluids in den menschlichen oder tierischen Körper mit einer Pumpe (7) zur Förderung des Fluids mit einer Durchflussrate (flow) und einer Steuereinrichtung (9) zur Steuerung der Pumpe (7) und zum Einstellen einer gewünschten Durchflussrate (Soll-flow) des Fluids sowie einer mit der Steuereinrichtung gekoppelten Druckmesseinrichtung (8) zur Erfassung des Drucks des von der Pumpe geförderten Fluids, wobei der Steuereinrichtung (9) ein Solldruck (pₛₒₗₗ) vorgegeben ist, **dadurch gekennzeichnet, dass** die Steuereinrichtung (9) den von der Druckmesseinrichtung (8) erfassten Druck (p) des Fluids mit dem Solldruck (pₛₒₗₗ) vergleicht und bei Überschreiten des Solldrucks über einen Zeitraum (Δt = t₂ - t₁) den zeitlichen Druckverlauf (p(t)) des von der Druckmesseinrichtung (8) erfassten Drucks (p) über diesen Zeitraum (Δt) zur Ermittlung eines Druckintegrals (I) integriert und die Pumpe (7) abschaltet oder mit geringerer Pumpleistung betreibt, wenn das ermittelte Druckintegral (I) einen vorgegebenen Integralgrenzwert (Iₘₐₓ) überschreitet.

2. Injektor nach Anspruch 1, **dadurch gekennzeichnet, dass** die Steuereinrichtung (9) den Differenzdruck (Δp = p - pₛₒₗₗ) zwischen dem von der Druckmesseinrichtung erfassten Druck (p) und dem Solldruck (pₛₒₗₗ) über den Zeitraum (Δt) zur Ermittlung des Druckintegrals (I) integriert.

3. Injektor nach Anspruch 1 oder 2, **dadurch gekennzeichnet, dass** die Durchflussrate (flow) des von der Pumpe (7) geförderten Fluids von der Steuereinrichtung (9) über eine Regelung der Pumpleistung auf den gewünschten Wert (Soll-flow) eingestellt wird.

4. Injektor nach Anspruch 3, **dadurch gekennzeichnet, dass** es sich bei der Pumpe (7) um eine von einem Elektromotor angetriebene Pumpe, insbesondere um eine Schlauchpumpe handelt und dass die Steuereinrichtung (9) die Motordrehzahl des Elektromotors regelt, um die Pumpleistung und dadurch die Durchflussrate (flow) des von der Pumpe (7) geförderten Fluids auf den gewünschten Wert (Soll-flow) einzustellen.

5. Injektor nach Anspruch 4, **wobei** die Durchflussrate (flow) direkt proportional zur Pumpleistung und insbesondere zur Motordrehzahl des Elektromotors ist.

6. Injektor nach einem der voranstehenden Ansprüche, **dadurch gekennzeichnet, dass** die Durchflussrate (flow) an der Steuereinrichtung auf einen gewünschten Durchflussratessollwert (Soll-flow) einstellbar ist und die Steuereinrichtung (9) die Pumpenleistung der Pumpe (7) so regelt, dass die tatsächliche Durchflussrate (flow) des von der Pumpe (7) geförderten Fluids auf den eingestellten Durchflussratesollwert (Soll-flow) gesteuert oder geregelt wird.

7. Injektor nach einem der voranstehenden Ansprüche, **wobei** die Pumpe (7), welche bevorzugt als Schlauchpumpe ausgebildet ist, das Fluid durch einen mit einem Fluidreservoir (2) in Verbindung stehenden Injektionsschlauch (3) fördert.

8. Injektor nach einem der voranstehenden Ansprüche, **dadurch gekennzeichnet, dass** der von der Druckmesseinrichtung (8) erfasste Druck (p) dem Druck des Fluids an einer von der Pumpe (7) entfernten Stelle, insbesondere stromabwärts der Pumpe (7), entspricht.

9. Injektor nach einem der voranstehenden Ansprüche, **wobei** die Druckmesseinrichtung den Druck (p) des Fluids unmittelbar oder mittelbar erfasst.

10. Injektor nach einem der voranstehenden Ansprüche, **dadurch gekennzeichnet, dass** die Steuereinrichtung (9) den von der Druckmesseinrichtung (8) erfassten Druck (p) des Fluids mit dem Solldruck (pₛₒₗₗ) vergleicht und bei Überschreiten des Solldrucks die Pumpleistung reduziert, bis der Druck (p) des Fluids wieder dem Solldruck (pₛₒₗₗ) entspricht.

## Claims

1. Injector for injecting a fluid into a human or animal body with a pump (7) for conveying the fluid at a throughflow rate (flow) and a control device (9) for controlling the pump (7) and for setting a required throughflow rate (set flow) of the fluid as well as a pressure-measuring device (8) coupled to the control device for recording the pressure of the fluid conveyed by the pump, wherein a set pressure (pₛₑₜ) is forwarded to the control device (9), **characterised in that** the control device (9) compares the pressure (p) of the fluid recorded by the pressure-measuring device (8) with the set pressure (pₛₑₜ) and on exceeding the set pressure over a period of time (Δt = t₂ - t₁), integrates the chronological pressure course (p(t)) of the pressure (p) recorded by the pressure-measuring device (8) over this period of time (Δt) to determine a pressure integral (I) and switches off the pump (7) or operates at lower pump throughput if the pressure integral (I) determined exceeds a predetermined integral limiting value (Imax).

2. Injector according to claim 1, **characterised in that** the control device (9) integrates the differential pressure (Δp = p - pₛₑₜ) between the pressure (p) recorded by the pressure-measuring device and the set pressure (pₛₑₜ) over the period of time (Δt) to determine the pressure integral (I).

3. Injector according to claim 1 or 2, **characterised in that** the throughflow rate (flow) of the fluid conveyed by the pump (7) is set to the required value (set flow) by the control device (9) via regulation of the pump throughput.

4. Injector according to claim 3, **characterised in that** the pump (7) is a pump driven by an electric motor, in particular a peristaltic pump, and **in that** the control device (9) regulates the motor speed of the electric motor in order to set the pump throughput and thus the throughflow rate (flow) of the fluid conveyed by the pump (7) to the required value (set flow).

5. Injector according to claim 4, wherein the throughflow rate (flow) is directly proportional to the pump throughput and in particular to the motor speed of the electric motor.

6. Injector according to one of the preceding claims, **characterised in that** the throughflow rate (flow) can be set to a required throughflow rate set value (set flow) at the control device and the control device (9) regulates the pump throughput of the pump (7) so that the actual throughflow rate (flow) of the fluid conveyed by the pump (7) is controlled or regulated to the set throughflow rate set value (set flow).

7. Injector according to one of the preceding claims, wherein the pump (7), which is preferably designed as a peristaltic pump, conveys the fluid through an injection tube (3) connected to a fluid reservoir (2).

8. Injector according to one of the preceding claims, **characterised in that** the pressure (p) recorded by the pressure-measuring device (8) corresponds to the pressure of the fluid at a point remote from the pump (7), in particular downstream of the pump (7).

9. Injector according to one of the preceding claims, wherein the pressure-measuring device records the pressure (p) of the fluid directly or indirectly.

10. Injector according to one of the preceding claims, **characterised in that** the control device (9) compares the pressure (p) of the fluid recorded by the pressure-measuring device (8) with the set pressure (pₛₑₜ) and on exceeding the set pressure, reduces the pump throughput until the pressure (p) of the fluid again corresponds to the set pressure (pset).

## Revendications

1. Injecteur servant à injecter un fluide dans l'organisme humain ou animal avec une pompe (7) servant à refouler le fluide à un débit (flow) et un dispositif de commande (9) servant à commander la pompe (7) et servant à régler un débit souhaité (flow théorique) du fluide et un dispositif de mesure de pression (8) couplé au dispositif de commande, servant à détecter la pression du fluide refoulé par la pompe, dans lequel une pression théorique (p_{théorique}) est spécifiée au dispositif de commande (9), **caractérisé en ce que** le dispositif de commande (9) compare la pression (p), détectée par le dispositif de mesure de pression (8), du fluide à la pression théorique (p_{théorique}) et intègre, en cas de dépassement de la pression théorique sur une durée de temps (Δt = t₂ - t₁), l'évolution de pression (p(t)) dans le temps de la pression (p) détectée par le dispositif de mesure de pression (8) sur ladite durée de temps (Δt) pour déterminer une intégrale de pression (I) et désactive la pompe (7) ou la fait fonctionner avec une puissance de pompage inférieure si l'intégrale de pression (I) déterminée dépasse une valeur limite d'intégrale (Iₘₐₓ) spécifiée.

2. Injecteur selon la revendication 1, **caractérisé en ce que** le dispositif de commande (9) intègre la pression différentielle (Δp = p - p_{théorique}) entre la pression (p) détectée par le dispositif de mesure de pression et la pression théorique (p_{théorique}) sur la durée de temps (Δt) pour déterminer l'intégrale de pression (I).

3. Injecteur selon la revendication 1 ou 2, **caractérisé en ce que** le débit (flow) du fluide refoulé par la pompe (7) est réglé par le dispositif de commande (9) par l'intermédiaire d'une régulation de la puissance de pompage sur la valeur souhaitée (flow théorique).

4. Injecteur selon la revendication 3, **caractérisé en ce que** la pompe (7) est une pompe entraînée par un moteur électrique, en particulier une pompe péristaltique, et que le dispositif de commande (9) régule la vitesse de rotation de moteur du moteur électrique pour régler la puissance de pompage et ainsi le débit (flow) du fluide refoulé par la pompe (7) sur la valeur souhaitée (flow théorique).

5. Injecteur selon la revendication 4, dans lequel le débit (flow) est directement proportionnel à la puissance de pompage et en particulier à la vitesse de rotation de moteur du moteur électrique.

6. Injecteur selon l'une quelconque des revendications précédentes, **caractérisé en ce que** le débit (flow) peut être réglé au niveau du dispositif de commande sur une valeur théorique de débit souhaitée (flow théorique) et le dispositif de commande (9) régule la puissance de pompage de la pompe (7) de telle sorte que le débit (flow) réel du fluide refoulé par la pompe (7) est commandé ou régulé sur la valeur théorique de débit (flow théorique) réglée.

7. Injecteur selon l'une quelconque des revendications précédentes, dans lequel la pompe (7), qui est réalisée de manière préférée sous la forme d'une pompe péristaltique, refoule le fluide par un tuyau flexible d'injection (3) en liaison avec un réservoir de fluide (2).

8. Injecteur selon l'une quelconque des revendications précédentes, **caractérisé en ce que** la pression (p) détectée par le dispositif de mesure de pression (8) correspond à la pression du fluide au niveau d'un emplacement éloigné de la pompe (7), en particulier en aval de la pompe (7).

9. Injecteur selon l'une quelconque des revendications précédentes, dans lequel le dispositif de mesure de pression détecte directement ou indirectement la pression (p) du fluide.

10. Injecteur selon l'une quelconque des revendications précédentes, **caractérisé en ce que** le dispositif de commande (9) compare la pression (p), détectée par le dispositif de mesure de pression (8), du fluide à la pression théorique (p_{théorique}) et réduit en cas de dépassement de la pression théorique la puissance de pompage jusqu'à ce que la pression (p) du fluide corresponde à nouveau à la pression théorique (p_{théorique}).
